(19) Europäisches Patentamt
European Patent Office
Office européen des brevets

(11) **EP 2 582 284 B1**

(12) **EUROPÄISCHE PATENTSCHRIFT**

(45) Veröffentlichungstag und Bekanntmachung des Hinweises auf die Patenterteilung:
**02.08.2023  Patentblatt 2023/31**

(21) Anmeldenummer: **11741087.8**

(22) Anmeldetag: **27.05.2011**

(51) Internationale Patentklassifikation (IPC):
***A61B 3/113*** *(2006.01)*

(52) Gemeinsame Patentklassifikation (CPC):
**A61B 3/113**

(86) Internationale Anmeldenummer:
**PCT/DE2011/001123**

(87) Internationale Veröffentlichungsnummer:
**WO 2011/157254 (22.12.2011 Gazette 2011/51)**

(54) **VERFAHREN UND VORRICHTUNG ZUR BESTIMMUNG DER AUGENPOSITION**

METHOD AND DEVICE FOR DETERMINING EYE POSITION

PROCÉDÉ ET APPAREIL DE DÉTERMINATION DE LA POSITION OCULAIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.06.2010   DE 102010024407**

(43) Veröffentlichungstag der Anmeldung:
**24.04.2013   Patentblatt 2013/17**

(73) Patentinhaber: **Chronos Vision GmbH**
**12247 Berlin (DE)**

(72) Erfinder: **SPASOVSKI, Saso**
**13088 Berlin (DE)**

(74) Vertreter: **Willems, Volker**
**Patentanwälte Weisse, Moltmann & Willems PartGmbB**
**Am Lomberg 13**
**42555 Velbert (DE)**

(56) Entgegenhaltungen:
| | |
|---|---|
| **WO-A1-00/27273** | **WO-A1-01/34021** |
| **WO-A1-99/18868** | **WO-A2-01/89373** |
| **DE-A1- 19 719 695** | **DE-A1-102006 002 001** |
| **US-A- 5 471 542** | **US-A- 6 019 755** |
| **US-A1- 2001 022 648** | **US-A1- 2006 215 111** |

**Beschreibung**

Die Erfindung betrifft ein Verfahren und eine Vorrichtung zur Bestimmung der Augenposition und/oder der Blickrichtung.

Hintergrund der Erfindung

Die Bestimmung der Augenposition und Blickrichtung ist für verschiedenste Anwendungen von Bedeutung, beispielsweise im Bereich der Medizintechnik. Bei der laserbasierten Augenoperation oder Laserablation, bei der mittels Laserpulsen Hornhautgewebe des Auges abgetragen wird, muss die genaue Augenposition bekannt sein. Aber auch im Bereich der Gleichgewichtsforschung ist die Ermittlung der Augenposition wesentlich, ebenso wie bei der Weiterentwicklung der Schnittstellen zwischen Mensch und Maschine, beispielsweise im Bereich der Fahrzeugsteuerung. Im Bereich der Werbung und der visuellen Kommunikation ist die Kenntnis der Blickrichtung des Betrachters ein wichtiger Faktor zur Beurteilung der Qualität von Grafiken, beispielsweise im Hinblick auf Kaufanreize, die auf Webseiten, Plakaten und Videos angeboten werden.

Zur Messung und Nachverfolgung der Augenposition werden sogenannte "eye tracker" eingesetzt. Bei einer Laserbehandlung wird damit die korrekte Ausrichtung eines Laserstrahls bestimmt, um die genaue Position der Gewebeablation zu gewährleisten. Dies ist erforderlich, da während einer Behandlung schnelle Augenbewegungen erfolgen, die einen Positionsfehler der Laserpulse relativ zum Auge zur Folge haben können. Das sogenannte Pupil Tracking liefert die laterale Position der Pupille (x-, y-Richtung), während ein Cyclotorsionstracker die Verdrehung des Auges um die Blickrichtung (z-Richtung) bestimmt.

Die Druckschrift DE102004020356 A1 beschreibt ein Verfahren zur Erfassung von Augenbewegungen, bei dem mit einem optoelektronischen Sensor Bildfolgen eines Auges erfasst werden. Dabei wird ein Bereich des Kopfes mit Markierungen versehen, die ebenfalls erfasst werden. Aus den gewonnenen Bilddaten wird unter Berücksichtigung des Pupillenmittelpunktes und der Markierungen am Kopf die Bewegung des Auges im Kopf ermittelt.

Die Patentschrift DE19954047B4 zeigt ein Verfahren zur Messung von Augenbewegungen, bei dem nichttoxische Tinktur-Markierungen in einer vorgegebenen Geometrie auf die Bindehaut des Auges aufgebracht werden und mit einem optoelektronischen Sensor Bildsequenzen des Auges erfasst werden. Daraus werden Koordinaten eines rotationsneutralen Bezugspunktes ermittelt, die mit den Koordinaten der Markierungen in Beziehung gesetzt werden, um daraus die Augenposition zu ermitteln.

Die Druckschrift DE102008049846A1 zeigt ein weiteres Verfahren zur Messung der Augenbewegung. Dabei wird die Lederhaut des Auges mit kohärentem Licht derart beleuchtet, dass durch interferierendes, an der Lederhaut gestreutes Licht, ein Fleckmuster entsteht. Anhand zweier Ausschnittsbilder wird eine Verschiebung des aufgenommenen Fleckmusters ermittelt und daraus die Augenbewegung bestimmt.

Die Druckschrift DE102008039838A1 beschreibt für Anwendungen in der Medizintechnik ein Verfahren zum Abtasten der dreidimensionalen Oberfläche eines Objekts mittels eines Lichtstrahl-Scanners, bei dem das Triangulationsprinzip angewandt wird. Wird ein Muster auf die Objektoberfläche projiziert, so lässt sich die Abstandsinformation zu allen Punkten des Musters in einem Kamerabild berechnen.

Das US Patent Nr. 4,761,071 zeigt ein Gerät und ein Verfahren zur Bestimmung der Topographie der Kornea und der Sklera des Auges durch Triangulation. Dabei wird eine fluoreszierende Substanz in das Auge gebracht, und durch einen einfallenden Lichtstrahl beleuchtet. Die Fluoreszenzstrahlung wird aus einer anderen Richtung erfasst und durch Triangulation wird die Erhöhung der Augenoberfläche bestimmt.

Nachfolgend werden weitere Dokumente kurz angeführt:
US 5, 471, 542 beschreibt eine Vorrichtung zur Bestimmung eines Sichtvektors für ein Auge. Die Vorrichtung umfasst eine Videokamera, die so ausgerichtet ist, dass sie ein Auge einer Bedienungsperson beobachtet, um ein Bild des Auges zu erhalten; eine Kameraschnittstellenelektronik und Bildprozessor zum Digitalisieren des Bildes des Auges des Bedieners und zum Analysieren des digitalisierten Bildes, um daraus eine Vielzahl von Punkten zu bestimmen, die die Iridellipse bestimmen, wobei die Punkte auf der Grenze zwischen der Sklera des Auges und der Iris liegen, dargestellt durch verschiedenes Lichtintensitäten; und einen Prozessor zum Verwenden der Informationen und einer Schätzung des Durchmessers der Iris beim Formulieren eines geschätzten optischen Achsenvektors für das Auge.

WO 01/34021 A1 beschreibt ein Verfahren und eine Anordnung zum Erfassen mehrdimensionaler Augenbewegungen und eine Färbetinktur dafür. Bei dem Verfahren erfolgen folgende Schritte: a) Auftragen von mindestens zwei ungiftigen Tinkturfarben nach vorgegebener Geometrie auf die Bindehaut des Auges außerhalb der Hornhaut, b) eindeutige Bestimmung der Koordinaten eines drehungsneutralen Bezugspunktes als Bezugsposition vor Beginn des Messvorgangs; c) Erfassung, Umwandlung in digitale Form und Speicherung der Bildsequenzen unter Verwendung des optoelektronischen Sensors d) Umwandlung in binäre Form und Unterteilung jedes Augenbildes in Segmente durch Trennen der Schwellenwerte, basierend auf der künstlichen Kontrastmarkierung und Bestimmung der Koordinaten der Flecken gemäß Schritt b); Zuordnung der Koordinaten des drehneutralen Bezugspunktes zu den Koordinaten der Flecken und Aufziehen einer Fläche über jedes segmentierte Augenbild; f) Bestimmung der Schwerpunkte der im Abstand voneinander aufgebrachten Flecken gemäß Schritt a) und Bestimmung der Abweichung von den Koordinaten des rekonstruierten Referenzpunktes gemäß Schritt b) als Maß für die Horizontale, vertikale und Torsionsposition des Auges.

DE 10 2006 002 001 A1 beschreibt ein Verfahren zur Bestimmung der räumlichen Relation eines Auges einer Person bezüglich einer Kameravorrichtung, die Bilder des Auges liefert. Das Verfahren umfasst eine Modellerstellungsphase, in der ein individuell angepasstes Modell des Auges konstruiert und eine räumliche Referenzrelation des Augenmodells bezüglich der Kameravorrichtung unter Verwendung eines Referenzbilds des Auges bestimmt wird; und eine Verfolgungsphase, in der Positions- und/oder Rotationskoordinaten des Auges durch Anpassen des Augenmodells an ein momentanes Bild des Auges bestimmt werden.

WO 00/27273 A1 beschreibt ein Verfahren zum Bestimmen der Position oder Haltung eines Auges, umfassend die Schritte des Richtens einer Vielzahl von augensicheren Lichtstrahlkomponenten auf das Auge, wobei die Komponenten einen Einfallsbereich auf das Auge definieren, der wesentlich größer als die Pupille ist; Empfangen eines Lichtbildes, das danach vom Auge reflektiert wird; Analysieren des Bildes durch Identifizieren, welche der Lichtstrahlkomponenten eine helle Augenreflexion in dem Bild erzeugt; und Bestimmen der Position oder Haltung des Auges durch weiteres Analysieren des Bildes auf der Grundlage der Identifizierung.

WO 99/18868 A1 beschreibt das Ableiten der relativen Position eines Auges durch Verfolgen einer Grenze des Auges, wie beispielsweise des Limbus. Es erfolgt das Verfolgen der Position des Auges zwischen der Sklera und der Iris und das Empfangen von reflektiertem Licht aus diesem Bereich. Die Intensität des reflektierten Lichts wird gemessen, um eine relative Position des Auges zu bestimmen.

DE 197 19 695 A1 beschreibt ein Verfahren und eine Vorrichtung zum Erfassen der Aufmerksamkeit von Lebewesen, insbesondere Menschen, innerhalb einer Vielzahl von analogen Lebewesen. Dazu wird Licht in Richtung der Gruppe emittiert. Das vom lebenden Organismus reflektierte Licht wird in Form eines Bildes erfasst. Das Bild wird so analysiert, dass das vom Augenhintergrund der Lebewesen reflektierte Licht gefiltert und als Bildpunkt erkannt wird. Die Bildpunkte werden gezählt.

US 2006/215111 A1 beschreibt ein Refraktionsmessinstrument zum Messen der Refraktion eines zu untersuchenden Auges, während die Person ein externes Objekt in einer natürlicheren Haltung betrachtet. Ein Messlichtstrahl von einer Lichtquelle wird von einem Spiegel reflektiert, zu einem Strahl mit Ringquerschnitt geformt, auf ein Prisma mit frei gekrümmter Oberfläche entlang einer optischen Achse gerichtet, von einer Oberfläche reflektiert, entlang einer optischen Achse zusammen mit dem sichtbaren Licht von außerhalb zu einem Auge geführt, und es wird ein Ringmuster auf dem Fundus gebildet. Der vom Fundus F reflektierte Messstrahl wird durch das Prisma mit frei gekrümmter Oberfläche und ein Prisma geführt und empfangen, wobei ein Ringmuster abgebildet wird. Eine Berechnungssteuervorrichtung analysiert das abgebildete Ringmuster und berechnet die Sphärizität, den Grad des Astigmatismus und den astigmatischen Achsenwinkel. Zur Messung trägt der Proband das Refraktionsmessgerät am Kopf durch einen Trageabschnitt

US 6 019 755 A beschreibt eine nicht-mechanische Entfernung des Epithels im Wesentlichen nur aus dem zu behandelnden Bereich der Hornhaut. Dabei bestrahlt ein Epithel ablatives Lasergerät den ausgewählten Bereich des Epithels mit ablativer Laserenergie, ein spektroskopisches System überwacht die Ablation und bestimmt spektroskopisch, ob Epithel abgetragen wird, und ein Kontrollsystem beendet die Epithelentfernung bei spektroskopischer Bestimmung einer fehlenden Epithelablation.

US 2001/022648 A1 beschreibt ein Verfahren zum optischen Verfolgen einer Bewegung eines Objekts, mit den Schritten: Auswählen einer Referenzmarke auf einem Objekt, wobei sich erste und zweite Oberflächenbereiche auf gegenüberliegenden Seiten der Referenzmarke auf dem Objekt in einer optischen Eigenschaft unterscheiden; wiederholtes Abtasten eines optischen Prüfstrahls vom ersten Oberflächenbereich zum zweiten Oberflächenbereich über die Referenzmarke mit einer ausgewählten Abtastgeschwindigkeit entlang einer ausgewählten Richtung, so dass sich eine Eigenschaft eines gestreuten Prüfstrahls, der von dem Objekt gestreut wird, aufgrund der Änderung der optischen Eigenschaft in den ersten und zweiten Oberflächenbereichen ändert, wenn der Sondenstrahl die Referenzmarke passiert; Erfassen eines Zeitpunkts, zu dem die Änderung der Eigenschaft des gestreuten Sondenstrahls auftritt, wenn der Sondenstrahl über die Referenzmarke abtastet; Bestimmen einer Zeitdifferenz zwischen der Zeit und einer Referenzzeit; und Verwenden der Zeitdifferenz, um einen Bewegungsbetrag des Objekts entlang der ausgewählten Richtung zu bestimmen.

WO 01/89373 A2 beschreibt ein Verfahren zum objektiven Messen der Augenausrichtung konsistent über und innerhalb von mehr als einem ophthalmologischen Gerät durch Durchführen eines Vergleichsprozesses, einschließlich: A. Identifizieren des Auges und der Position des Auges an jedem Gerät relativ zu einem geräteabhängigen Koordinatensystem; B. Verwenden von räumlich definierten Eigenschaften des Auges, um die Position eines Koordinatensystems relativ zum Auge zu fixieren, um ein augenabhängiges Koordinatensystem zu erhalten; und C. Verwenden des augenabhängigen Koordinatensystems zum Berechnen einer Koordinatentransformation zwischen geräteabhängigen Koordinatensystemen und dem augenabhängigen Koordinatensystem.

Bei den meisten bekannten Eye-Tracking-Systemen wird die Position des Auges mittels aufgenommener Kamerabilder in einer Ebene erfasst, die üblicherweise in der zur transversal-vertikalen Körperebene parallelen Augenebene liegt (x/y-Ebene). Zur vollständigen Bestimmung der Augenposition ist jedoch neben der Kenntnis der lateralen Position der Pupille (x/y-Position) und der Verdrehung des Auges um die Blickrichtung (z-Richtung) die Ermittlung der Verkippung des Augapfels von Bedeutung. Wenn die Verkippung außer Acht gelassen wird, tritt ein Dezentrierungsfehler DE an der Augenoberfläche auf.

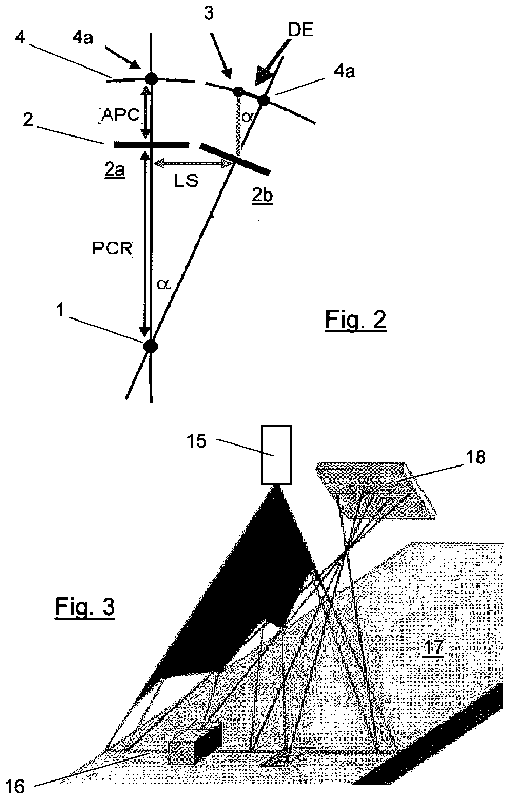

Der Dezentrierungsfehler ist in **Fig. 2** gezeigt. Dabei ist eine Pupille 2 eines Auges und die darüber im Abstand APC befindliche Hornhaut 4 schematisch in zwei Positionen gezeigt, nämlich zunächst in einem ersten Zustand 2a ohne Verkippung, sowie weiterhin in einem zweiten Zustand 2b, indem sie im Bild nach rechts hin verkippt ist. Der Winkel $\alpha$ beschreibt den Winkel der Verkippung gegenüber dem Normalzustand, in dem das Auge nicht verkippt ist. Die Pupille 2 befindet sich in einem Abstand PCR vom Rotationszentrum 1 des Auges. Im nicht verkippten ersten Zustand 2a befindet sich ein zentraler Punkt 4a der Hornhaut 4 exakt oberhalb des Zentrums der Pupille 2. Bei einer Bilderfassung des Auges aus der Blickrichtung liegt der Punkt 4a der Hornhaut 4 an derselben Stelle im Bild wie das Pupillenzentrum. Bei einer Verkippung des Auges um das Rotationszentrum 1, ergibt sich in dem erfassten Bild des Auges eine laterale Verschiebung LS des Zentrums der Pupille 2. Jedoch liegt der zentrale Punkt 4a der Hornhaut 4 in dem erfassten Bild des Auges nicht mehr an derselben Stelle wie das Pupillenzentrum, sondern ist um einen Abstand davon entfernt, der den Dezentrierungsfehler DE bildet.

Wird zum Beispiel im Fall einer Laserablation der Punkt 4a als ein vorbestimmtes Ablationszentrum entsprechend der lateralen Verschiebung LS nachgeführt, ergibt sich auf der Hornhaut 4 ein aktuelles Ablationszentrum 3, das gegenüber dem vorbestimmten Ablationszentrum 4a um den Dezentrierungsfehler DE verschoben ist. Dadurch ergibt sich eine Ablation, die trotz einer Nachführung von der Sollposition abweicht. Will man den Dezentrierungsfehler DE berücksichtigen, so muss die Verkippung des Auges bekannt sein.

Die Kenntnis des Verkippungswinkels ist eine notwendige Bedingung, für die Korrektur des Fehlers DE aber nicht hinreichend. Für eine exakte Korrektur müssen daher weitere Parameter bekannt sein: der Abstand APC zwischen Pupille 2 und Hornhaut 4, sowie der Abstand PCR zwischen dem Rotationszentrum 1 und der Pupille 2. Werden für diese Parameter statistische Mittelwerte angesetzt, dann wird der Fehler DE mindestens verringert. Meist sind die fehlenden Parameter jedoch aus Voruntersuchungen bekannt.

Bei der Laserablation ist die Korrektur der Behandlungsdezentrierung, die durch die Verkippung des Auges entsteht, umso wichtiger, je höher die Schussfrequenz ist. Geht man von einer Frequenz von ca. 100 Hz aus, so dauert eine typische Behandlung etwa 100s. Ist die Verkippung des Auges in dieser Zeit nach Richtung und Betrag normalverteilt, dann führt das dazu, dass die Behandlung "verschmiert". Das Abtragvolumen entspricht zwar ungefähr dem Soll, aber der Behandlungsdurchmesser steigt und folglich sinkt die erreichte Abtragtiefe in Abhängigkeit von der Standardabweichung der Verkippungsbewegung. Ist die Verkippung während der Behandlung nicht normalverteilt oder im Extremfall systematisch verkippt, dann wird der Abtrag für verschiedene Richtungen auch bei geringen Schussfrequenzen verschieden stark vom Sollabtrag abweichen und es kann zu induzierten Fehlsichtigkeiten kommen. Beträgt die Frequenz z.B. 1000Hz, so reduziert sich die Dauer einer typischen Behandlung auf 10s. Die Wahrscheinlichkeit, dass die Verkippung des Auges in dieser kurzen Zeitspanne normalverteilt ist, sinkt und kurze Abweichungen der Blickrichtung können drastische Folgen haben.

Zur Ermittlung der Verkippung des Auges wird in der Druckschrift DE102004025999A1 eine Vorrichtung gezeigt, die ein Referenzobjekt umfasst, das auf dem Auge positioniert wird. Eine Sensoreinrichtung erfasst das Referenzobjekt in zwei translatorischen Richtungen, und drei weitere Sensoren erfassen den jeweiligen Abstand zu drei Abtastpunkten auf dem Referenzobjekt. Eine Rechnereinheit ermittelt in Echtzeit die Lage des Referenzobjekts. Diese Vorrichtung hat jedoch den Nachteil, dass das Referenzobjekt in Form eines Ringes konzentrisch zur Pupille auf das Auge aufgesetzt werden muss.

Zusammenfassung der Erfindung

Es ist die Aufgabe der Erfindung, ein Verfahren anzugeben, mit dem eine möglichst genaue und schnelle Messung der Augenposition und/oder der Blickrichtung unter Berücksichtigung der Verkippung des Auges erreicht wird. Weiterhin soll eine Vorrichtung geschaffen werden, mit der die Augenposition oder die Blickrichtung sowie die Verkippung des Auges genau und schnell bestimmt werden kann.

Diese Aufgabe wird gelöst durch das Verfahren zur Bestimmung der Augenposition und/oder der Blickrichtung gemäß Patentanspruch 1, und durch die Vorrichtung gemäß Patentanspruch 15. Weitere vorteilhafte Merkmale ergeben sich aus den abhängigen Ansprüchen, der Beschreibung und den Zeichnungen.

Die Erfindung schafft ein Verfahren zur Bestimmung der Augenposition, bei dem Punkte der Übergangsregion zwischen Sklera und Iris des Auges ermittelt werden, deren Koordinaten berechnet werden, und daraus die Lage der Irisebene oder einer parallel dazu ausgerichteten Ebene bestimmt wird, wobei ein Lichtmuster auf eine Oberfläche des Auges projiziert und dort abgebildet wird, wobei das Lichtmuster die Übergangsregion zwischen Sklera und Iris schneidet, und ein Bild des auf der Augenoberfläche abgebildeten Lichtmusters erfasst wird, aus dem die räumlichen Koordinaten der Punkte der Übergangsregion zwischen Sklera und Iris durch Triangulation oder durch Stereovision ermittelt werden,

wobei aus den räumlichen Koordinaten der Punkte die Lage der Irisebene oder einer parallel dazu ausgerichteten Ebene bestimmt wird.

Dadurch ist es möglich, die Augenposition, die Blickrichtung und insbesondere auch die Verkippung des Auges sehr schnell und mit hoher Genauigkeit zu bestimmen, wobei vorher keine Objekte auf dem Auge platziert werden müssen.

Mit dem erfindungsgemäßen Verfahren besteht weiterhin die Möglichkeit der Ermittlung der lateralen Pupillenposition. Das Lichtmuster verschwindet in der Pupille, das heißt, es wird abrupt unterbrochen. Aus der abrupten Unterbrechung des Musters lässt sich der Pupillenrand ermitteln.

Das Lichtmuster kann auch durch Verwendung eines Gitters oder Reflexionsgitters oder durch diffraktive Elemente auf der Augenoberfläche erzeugt werden.

Die Koordinaten der Punkte (P1, P2, P3, P4) werden durch Triangulation oder durch Stereovision berechnet. Triangulation hat bei dem Verfahren den Vorteil einer besonders hohen Geschwindigkeit. Bei Bestimmung der Koordinaten mittels Stereovision werden eine oder mehrere zusätzliche Kameras verwendet. Denkbar ist auch die Verwendung einer Kamera und mehrerer Spiegel und/oder Prismen. Oder man benutzt nur eine Kamera und nur einen zusätzlichen Spiegel: die (einzige) Kamera beobachtet die Szene mit z.B. der linken Sensorhälfte direkt und mit der anderen Hälfte den Spiegel, der die Szene aus einem anderen Winkel zeigt. Es ist auch möglich, mit Hilfe von mindestens zwei Bildleitern, die die Szene aus verschiedenen Blickwinkeln auf den Kamerachip abbilden und einer Kamera ebenfalls Stereovision zu verwenden.

Eine weitere vorteilhafte Möglichkeit zur Bestimmung der Koordinaten der Punkte ist das sogenannte "depth from focus" Verfahren. Dabei fährt man die Schärfebene des Objektivs gezielt durch das Messvolumen. Es sind immer nur die Teile des Lichtmusters, die sich in der aktuellen Schärfeebene befindet, kontrastreich.

Vorteilhafterweise werden die Punkte im Bild derart ermittelt, dass sie sich auf der Sklera in unmittelbarer Nähe zur Iris befinden. Dadurch ergibt sich eine noch höhere Genauigkeit, da das Licht nicht an Grenzflächen innerhalb des Auges gebrochen wird. Mindestens drei Punkte sind nötig, wobei es nach oben keine Grenze gibt. Je mehr Punkte bestimmt werden, umso genauer wird das Ergebnis.

Bevorzugt werden die Punkte aus Unstetigkeiten des abgebildeten Lichtmusters an der Grenze zwischen Sklera und Iris ermittelt. Dadurch können exakt die Punkte ermittelt werden, an denen das Lichtmuster die Übergangsregion zwischen Sklera und Iris schneidet.

Ist die Unstetigkeit zum Beispiel aus anatomischen Gründen zu gering, dann kann auch nach einem Helligkeitsgradienten entlang der Linien gesucht werden, denn die Linien sind im Allgemeinen auf der Sklera heller. Es ist auch denkbar, dass mittels Bildverarbeitungsverfahren der Limbus im Kamerabild gesucht wird. Die Punkte werden dann aus dem Schnitt der Linien mit dem Limbus ermittelt.

Vorteilhafterweise wird das auf dem Auge erzeugte Lichtmuster aus einzelnen Linien bzw. Linienfragmenten gebildet, deren virtueller Schnitt mit dem Limbus des Auges durch eine approximierende Funktion berechnet wird, um die Punkte zu identifizieren. Dies hat den Vorteil, dass kein Teil des Lichtmusters auf die Iris projiziert wird, so dass während der Messung kein Licht ins Auge gelangt.

Bevorzugt wird das Lichtmuster unter einem Einfallswinkel auf das Auge projiziert bzw. abgebildet. Durch einen schräg einfallenden Abbildungsstrahl oder Projektionsstrahl zur Projektion des Lichtmusters auf dem Auge ergibt sich eine sehr große Genauigkeit bei der Anwendung des Triangulations- bzw. Lichtschnittverfahrens zur Bestimmung der Lage der Orte, an denen das Lichtmuster reflektiert wird.

Das Lichtmuster kann insbesondere aus Lichtstreifen oder Lichtringen gebildet sein, wobei die Lichtstreifen z. B. parallel und die Lichtringe z. B. konzentrisch zueinander ausgerichtet sein können. Das Lichtmuster kann aber auch aus Lichtstreifen in Form eines Kreuzes gebildet sein. Dadurch ergibt sich eine hohe Flexibilität in Abhängigkeit vom jeweiligen Zweck der Messung so wie, je nach Anwendungsfall, eine Optimierung der Messgenauigkeit.

Vorteilhafterweise kann das Lichtmuster auch ein oder mehrer breite Streifen umfassen, die zum Beispiel scharf berandet sind, und nicht notwendigerweise rechteckig und parallel ausgebildet sein müssen.

Das Lichtmuster ist bevorzugt mehrfarbig und wird zum Beispiel mit einer Farbkamera erfasst. Dadurch steigt der Informationsgehalt der Bilder: die Bildverarbeitungseinheit kann z.B. die Streifen anhand der Farbe finden und voneinander unterscheiden. Verwendet man viele Streifen oder andere komplexe Muster, dann können Mehrdeutigkeiten bei der Streifenidentifizierung vermieden werden. Ein weiterer Vorteil besteht darin, dass sich die Absorptionseigenschaften von weißer Sklera und farbiger Iris unterscheiden. Die von der Sklera gestreuten oder reflektierten farbigen Streifen weisen nach Aufnahme mittels einer Kamera eine andere farbliche Zusammensetzung auf als die von der Iris kommenden. Das kann bei der Suche nach dem Limbus ausgenutzt werden. Hier sind merkliche Gradienten des Farbverlaufs entlang der Linien zu erwarten.

Bevorzugt werden der Grad und die Richtung einer Verkippung des Auges aus dem Normalenvektor einer durch die Punkte aufgespannte Ebene ermittelt.

Eine Kalibrierung erfolgt zum Beispiel dadurch, dass das Lichtmuster auf eine ebene Fläche projiziert wird, die während des Kalibriervorgangs in Richtung der optischen Achse einer Kamera oder Bilderfassungseinrichtung verschoben wird. Die ebene Fläche wird in Richtung der z-Achse verschoben. Dadurch können Verschiebungen des Lichtmusters im aufgenommenen Bild in eine sehr genaue Beziehung zur Höhenlage und Höhenstruktur der Fläche gebracht werden, auf denen das Lichtmuster während der Messung abgebildet ist. Die Verschiebung erfolgt nicht notwendigerweise in Richtung der Kamera.

Während der Messung kann das Lichtmuster über das Auge bewegt werden, oder es können zeitlich veränderliche Lichtmuster erzeugt werden. Dabei können z. B. die Linien des Lichtmusters derart bewegt werden, dass sie stets optimal zum Limbusrand positioniert sind. Bei einer Bewegung des Lichtmusters kann als Muster z. B. auch nur eine Linie verwendet werden. Zwischen zwei Kameraaufnahmen hat sich dann die Linie ausreichend weit bewegt, und gleichzeitig ist die Bewegung des Auges bei entsprechend hoher Geschwindigkeit vernachlässigbar.

Da beide Augen, wenn man sich auf einen relativ fernen Punkt fixiert, in die gleiche Richtung schauen, kann das Lichtmuster zum Beispiel auch auf das eine Auge projiziert werden, um die Blickrichtung oder Augenposition des anderen Auges zu ermitteln. Das ist vorteilhaft, da ein durch refraktive Chirurgie behandeltes Auge eine extrem rauhe Oberfläche aufweist und dadurch die Ergebnisse beeinflussen kann.

Vorteilhafterweise befinden sich beide Augen im Blickfeld der Kamera, selbst wenn nur ein Auge vermessen wird. Dadurch kann sichergestellt werden, dass das richtige Auge behandelt wird. Vorteilhafterweise beträgt das Seitenverhältnis des Sensors der Kamera 3:1. Dadurch können beide Augen ohne Auflösungsverlust aufgenommen werden.

Aus dem Winkel der Verbindungslinie beider Pupillenmitten mit einer konstanten Referenzlinie kann eine Verdrehung des Kopfes bzw. die Kopftorsion ermittelt werden.

Die erfindungsgemäße Vorrichtung zur Erfassung der Augenposition umfasst eine Bilderfassungseinrichtung und eine Bildverarbeitungseinheit zur Ermittlung mehrerer Punkte, an denen die Sklera des Auges an die Iris grenzt, wobei die Bildverarbeitungseinheit Koordinaten der ermittelten Punkte berechnet und daraus die Lage der Irisebene oder einer parallel dazu ausgerichteten Ebene ermittelt, und weiterhin eine Einrichtung zur Projektion und Abbildung eines Lichtmusters auf einer Oberfläche des Auges, und eine Bilderfassungseinrichtung zur Erfassung des auf der Oberfläche des Auges abgebildeten Lichtmusters, wobei die Bildverarbeitungseinheit aus dem erfassten Lichtmuster durch Triangulation oder durch Stereovision die räumlichen Koordinaten der Punkte ermittelt, an denen das Lichtmuster die Übergangsregion zwischen Sklera und Iris schneidet, und daraus die Lage der Irisebene oder einer parallel dazu ausgerichteten Ebene ermittelt.

Die Bildverarbeitungseinheit kann zum Beispiel Teil der Kamera sein. Bestimmte Kameratypen benötigen keine externe Verarbeitungseinheit. Die Algorithmen zur Bildverarbeitung laufen in diesem Fall intern in einem Prozessor und/oder direkt auf dem Sensor.

Die Bildverarbeitungseinheit kann insbesondere derart gestaltet sein, dass sie die Punkte derart ermittelt, dass sie auf der Sklera in unmittelbarer Nähe zur Iris liegen.

Vorteilhafterweise ist die Bildverarbeitungseinheit derart ausgestaltet, dass sie die Punkte aus Unstetigkeiten des projizierten Lichtmusters an der Grenze zwischen Sklera und Iris identifiziert. Die Punkte können aber auch aus Helligkeits- oder Farbgradienten in den Linien ermittelt werden.

Insbesondere kann die Einrichtung zur Erzeugung des Lichtmusters derart gesteuert sein, dass als Lichtmuster mehrere einzelne Linien bzw. Linienfragmente auf das Auge projiziert werden, wobei die Bildverarbeitungseinheit z. B. den virtuellen Schnitt der Linienfragmente mit dem Limbus des Auges durch eine approximierende Funktion berechnet, um die Punkte zu identifizieren.

Insbesondere kann ein Glasfaserbildbündel im Strahlengang vor der Bilderfassungseinrichtung angeordnet sein, um das projizierte Lichtmuster auf dem Auge zu erfassen. Dadurch kann entweder die Belichtungszeit der Kamera oder die Intensität des Mustergenerators verringert werden. Das Glasfaserbildbündel ist geordnet und dadurch für die Bildübertragung nutzbar.

Die Vorrichtung kann mit einer Steuereinrichtung zur Aussteuerung einer Laserablationseinheit versehen sein, die deren Ablationsstrahl bei einer Veränderung der Augenposition nachführt oder vom Auge ablenkt oder unterbricht. Dadurch werden Ablationsfehler aufgrund einer Veränderung der Augenposition vermieden.

Vorteilhafterweise ist ein Fluoreszenzlicht-Sensor zur Erfassung von Ablationspunkten auf dem Auge vorgesehen, die vom Ablationsstrahl getroffen wurden, wobei beispielsweise die Rechnereinheit die Ortskoordinaten der erfassten Ablationspunkte zum Beispiel durch Triangulation ermittelt. Dadurch kann z. B. im Fall einer Laserablation jeder einzelne Ablationsspot anhand des emittierten Fluoriszenlichtes registriert werden und der Schwerpunkt seiner Projektion auf der Sensorfläche mit hoher Frequenz bestimmt werden.

Insbesondere lassen sich per Triangulation alle drei Raumkoordianten der getroffenen Fläche ermitteln. In ihrer Summen ergibt sich eine Höhenkarte der äußeren Hornhautoberfläche.

Insbesondere ist die erfindungsgemäße Vorrichtung derart ausgestaltet, dass sie zur Durchführung des erfindungsgemäßen Verfahrens geeignet ist und entsprechende Merkmale aufweist.

Beschreibung bevorzugter Ausführungsbeispiele

Nachfolgend wird die Erfindung detailliert anhand von bevorzugten Ausführungsbeispielen unter Bezugnahme auf die Zeichnungen näher beschrieben. Merkmale, die im Zusammenhang mit dem Verfahren beschrieben werden gelten auch für die Vorrichtung und umgekehrt. Dabei zeigen:

| | |
|---|---|
| **Figur 1** | eine schematische Darstellung einer ersten bevorzugten Ausführungsform der erfindungsgemäßen Vorrichtung; |
| **Figur 2** | eine Verkippung des Auges und den daraus resultierenden Dezentrierungsfehler, wenn die Augenverkippung nicht berücksichtigt wird; |
| **Figur 3** | eine schematische Darstellung des Triangulationsverfahrens; |
| **Figuren 4a und 4b** | schematisch das Bild eines Auges, auf dem erfindungsgemäß parallele Lichtstreifen abgebildet sind; |
| **Figuren 5a bis 5c** | schematisch das Bild eines Auges mit einem darauf erfindungsgemäß projizierten statischen Kreuz; |
| **Figur 6** | schematisch das Bild eines Auges, auf dem Linien nur im Bereich der Sklera projiziert sind, |
| **Figur 7** | ein reales Bild eines Auges, auf das gemäß der Erfindung eine Lichtlinie abgebildet ist; |
| **Figur 8** | eine schematische Schnittansicht eines Auges im Bereich der Iris; |
| **Figuren 9a und 9b** | einen prinzipiellen Aufbau zur Kalibrierung der erfindungsgemäßen Vorrichtung und ein Kalibrierungsbeispiel; |
| **Figur 10** | eine zweite bevorzugte Ausführungsform der erfindungsgemäßem Vorrichtung mit einem Laser und einem diffraktiven optischen Element; |
| **Figur 11** | eine dritte bevorzugte Ausführungsform erfindungsgemäßem Vorrichtung mit einer Anzahl von Lasern als Projektionseinrichtung; |
| **Figur 12** | eine vierte bevorzugte Ausführungsform der erfindungsgemäßem Vorrichtung, mit der ein breiter Lichtstreifen erzeugt wird; |
| **Figur 13** | eine fünfte Ausführungsform der erfindungsgemäßem Vorrichtung mit einem Beamer als Projektionseinheit; |
| **Figur 14** | eine sechste bevorzugte Ausführungsform der erfindungsgemäßem Vorrichtung, mit der das Lichtmuster aus verschiedenen Richtungen auf das Auge projiziert wird; |
| **Figur 15** | eine siebte bevorzugte Ausführungsform der erfindungsgemäßem Vorrichtung, mit der ein Muster über das Auge bewegt wird; |
| **Figur 16** | eine achte bevorzugte Ausführungsform der erfindungsgemäßem Vorrichtung mit einem Glasfaserbündel vor der Kamera; |
| **Figur 17** | eine neunte bevorzugte Ausführungsform der erfindungsgemäßem Vorrichtung mit einem Fluoreszenzlichtsensor; und |
| **Figur 18** | verschiedene Lichtmuster, zur erfindungsgemäßen Abbildung auf dem Auge. |

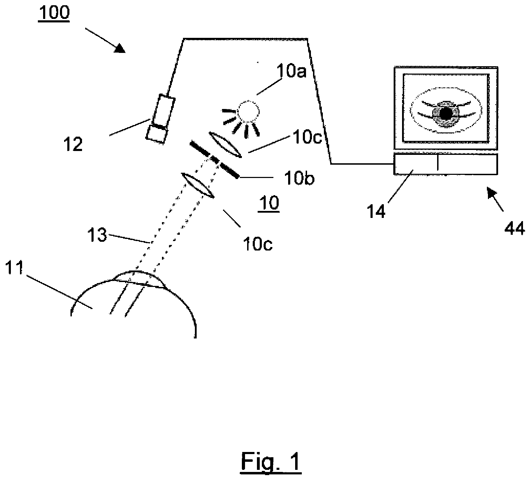

In **Figur 1** ist eine Vorrichtung 100 zur Bestimmung der Augenposition als bevorzugte Ausführungsform der Erfindung dargestellt. Eine Projektionseinrichtung 10, bestehend aus einer Lichtquelle 10a, einem Spaltelement 10b zur Erzeugung eines Doppelspalts, und einem Linsensystem 10c, dient zur Erzeugung eines Lichtmusters und dessen Projektion auf ein Auge 11 durch Lichtstrahlen 13. Eine Bilderfassungseinrichtung in Form einer Kamera 12 erfasst das auf dem Auge 11 abgebildete Lichtmuster. Dabei bildet die optische Achse der Kamera 12 zur Aufnahme des Bildes auf dem Auge 11 einen Winkel zum Strahlengang des Lichtstrahls 13 der Projektion auf das Auge 11. Die Kamera 12 ist elektrisch an eine Bildverarbeitungseinheit 14 gekoppelt, die im vorliegenden Fall Teil einer Rechnereinheit 44 ist. Die Bildverarbeitungseinrichtung kann aber auch an anderer Stelle realisiert sein, zum Beispiel in der Kamera selbst.

Durch den Winkel zwischen dem projizierten Lichtstrahl 13 und der Aufnahmerichtung der Kamera 12 lässt sich durch Anwendung des Triangulationsverfahrens bzw. Lichtschnittverfahrens ein Höhenprofil des auf dem Auge 11 abgebildeten Lichtmusters bestimmen.

**Figur 3** zeigt das Prinzip des Lichtschnittverfahrens bzw. Triangulationsverfahrens. Durch einen Laser 15 wird ein Lichtstreifen 16 als ein Muster auf die Oberfläche eines Objekts 17 projiziert. Ein Bildsensor 18 erfasst den auf der Oberfläche abgebildeten Lichtstreifen 16 aus einer Richtung, die sich von der Richtung des auf die Objektoberfläche 17 einfallenden Lichtstrahls des Lasers 15 unterscheidet. Durch den Winkel zwischen dem einfallenden Lichtstrahl und dem zum Bildsensor 18 hin reflektierten oder gestreuten Lichtstrahl lässt sich aus der Lage des Lichtstreifens 16 im aufgenommen Bild die Höhe der Objektoberfläche 17 im Bereich des Lichtstreifens 16 oder auch die jeweilige Entfernung zum Bildsensor 18 bestimmen.

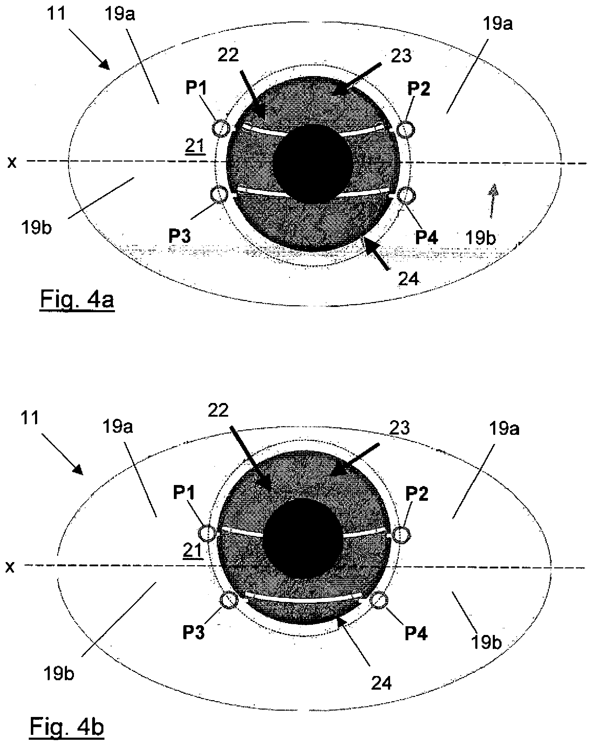

Die **Figuren 4a und 4b** zeigen das Auge 11 mit zwei darauf abgebildeten statischen Linien 19a, 19b, die ein Lichtmuster bilden. Dabei sind die Linien 19a, 19b bei der Projektion oder Abbildung parallel zueinander ausgerichtet, jedoch ist ihr Abbild auf dem Auge 11 aufgrund der Krümmung der Sklera 21 und der Brechung durch die Kornea 22 ebenfalls gekrümmt. Vom Betrachter bzw. der Kamera aus gesehen hinter der Kornea 22 befindet sich die Iris 23, die im wesentlichen planar ausgebildet ist, ebenso wie der Limbus 24. Während in Figur 4a das Auge 11 nicht verkippt ist, ist das in Figur 4b gezeigte Auge 11 nach oben hin verkippt. Dabei zeigt das verkippte Auge 11 eine Position mit Blickrichtung nach oben, was einer Verkippung des Auges um die x-Achse entspricht. Gleichzeitig ist in gegenüber der in Figur 4a dargestellten unverkippten Position eine laterale Verschiebung der Iris 23 nach oben, d.h. in y-Richtung, zu erkennen.

Am Übergang zwischen der Sklera 21 und der Kornea 22 sind die Linien 19a, 19b aufgrund des Höhenunterschiedes am Limbusrand jeweils unterbrochen und bilden an dieser Stelle eine Unstetigkeit.

Die Bildverarbeitungseinrichtung 14 (siehe Fig. 1) ist derart ausgestaltet, dass sie die Orte ermittelt, an denen die auf dem Auge 11 erzeugten Linien 19a, 19b Unstetigkeiten oder Helligkeits- oder Farbgradienten aufweisen. Dies geschieht z. B. dadurch, dass die hellsten Bereiche als Linien identifiziert werden und darin befindliche Dunkelbereiche als Unterbrechungen identifiziert werden. Aus der Lage der Unterbrechungen bzw. Unstetigkeiten der Linien 19a, 19b werden mittels Bildverarbeitung Punkte P1, P2, P3, P4 ermittelt, die sich auf den Linien 19a, 19b befinden und außerhalb der Iris 23 angeordnet sind, jedoch in deren unmittelbarer Nähe bzw. daran angrenzend.

Mit den Punkten P1, P2, P3, P4 lässt sich ein ebener Kreis im Raum $R^3$ berechnen. Die Normale der Kreis-Ebene stellt den Limbus- oder Irisnormalenvektor dar. Aus diesem lässt sich über das Skalarprodukt mit der z-Achse der Verkippungswinkel $\alpha$ bestimmen. Die Drehachse ist senkrecht zur Ebene, die durch die z-Achse und den Limbusnormalenvektor aufgespannt wird. Aus dem Zentrum des ebenen Kreises ergibt sich in drei Dimensionen das Limbuszentrum, welches z.B. beim eye tracking statt der Pupille verwendet werden kann.

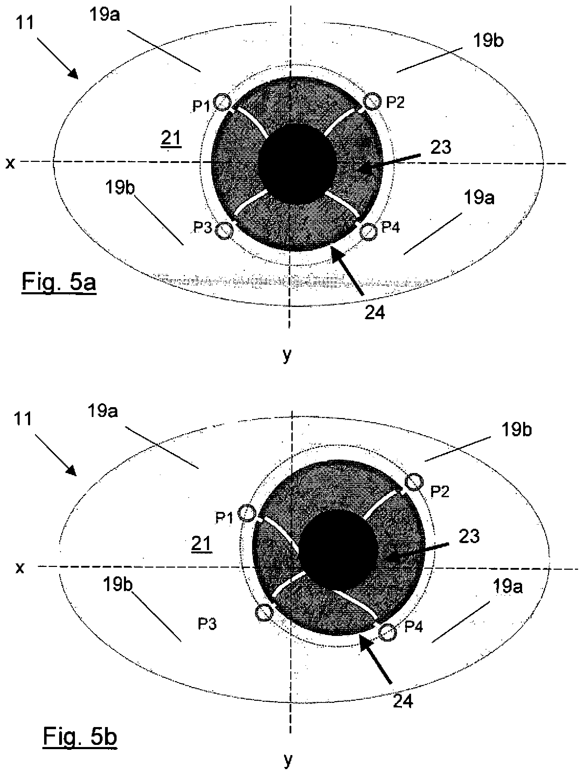

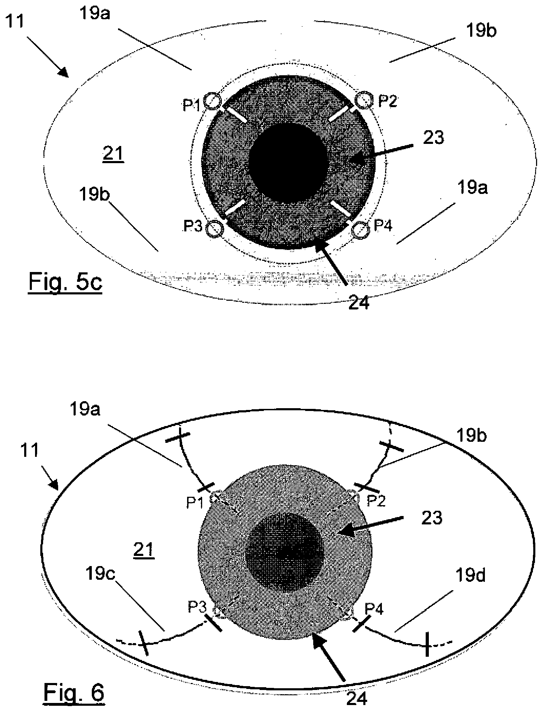

Die **Figuren 5a, 5b und 5c** zeigen schematisch eine Draufsicht auf das Auge 11, wobei die Linien 19a, 19b bei Ihrer Projektion als ein statisches Kreuzes geformt sind. Auf dem Auge erscheinen die Linien 19a, 19b gekrümmt, wie oben erläutert. Während in Figur 5a das Auge 11 in einer nicht verkippten Position dargestellt ist, ist in Figur 5b das Auge 11 nach rechts oben verkippt, Dass heißt, es liegt eine Verkippung sowohl um die x-Achse als auch um die y-Achse vor. Gleichzeitig sind Limbus 24 und Iris 23 im Bild lateral nach rechts oben bzw. in x- und y- Richtung verschoben. Bei dem in Figur 5c dargestellten Bild ist der zentrale Teil des Kreuzes nicht vorhanden. Die Projektion eines Musters ohne den zentralen Teil hat den Vorteil, dass mögliche Algorithmen, die die Iris 23 einbeziehen, weniger beeinflusst werden. Die Orte der Unstetigkeiten der Linien 19a, 19b werden durch die Bildverarbeitungseinrichtung 14 ermittelt und daraus die Punkte P1, P2, P3 und P4 bestimmt.

Die **Figur 6** zeigt ein Bild des Auges 11, bei dem die Linien 19a, 19b, 19c, 19d so kurz gehalten sind, dass sie nur auf der Sklera 21 abgebildet sind, nicht jedoch im Bereich der Iris 23. In der Figur sind die Anfangs- und Endpunkte der Linien 19a, 19b, 19c, 19d durch kurze Begrenzungsstriche markiert. Zur Bestimmung der Punkte P1, P2, P3, P4 werden die Linien 19a, 19b, 19c, 19d durch die Bildverarbeitungseinrichtung 14 (siehe Figur 1) mit einer approximierenden Funktion zu einem virtuellen Schnitt mit dem Limbus 24 verlängert. Dies hat den Vorteil, dass auf der Iris 23 kein Lichtmuster abgebildet wird. Dies ist natürlich auch mit anderen Mustern möglich, beispielsweise bei der Beleuchtung mit parallelen Linien.

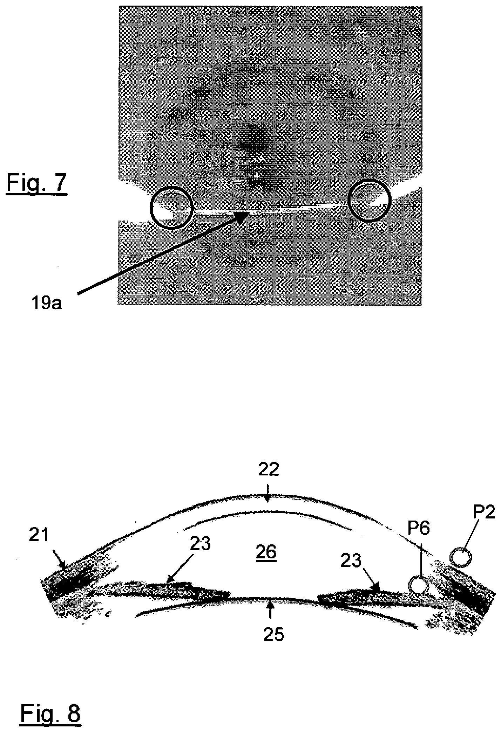

**Figur 7** zeigt eine reale Aufnahme des menschlichen Auges, mit einer auf das Auge projizierten Linie 19a, wobei die Unstetigkeiten der Linie 19a an dem Übergang zwischen Sklera 21 und Iris 23 gut zu erkennen sind.

**Figur 8** zeigt einen Schnitt durch das Auge im Bereich der Iris 23, mit der darüber befindlichen Kornea 22 und der Linse 25. Am äußeren Rand der Kornea 23 befindet sich die Sklera 21 außerhalb des Bereichs der Iris 23. In der Kammer 26 zwischen der Iris 23 und der Kornea 22 befindet sich Kammerwasser. Die Unstetigkeiten der projizierten Linien 19a, 19b, 19c, 19d (siehe Figuren 4 bis 6) sind durch den Höhenunterschied zwischen Iris und Limbus bedingt. In der Figur ist eine Unstetigkeit durch die Punkte P2 und P6 gekennzeichnet, die Endpunkte einer abgebildeten Linie an deren Unterbrechung darstellen.

Bei der Durchführung des Verfahrens zur Bestimmung der Augenposition beobachtet die in **Figur 1** gezeigte Kamera 12 die Szene und ein in der Bildverarbeitungseinrichtung 14 realisierter Algorithmus erkennt die Linien, die durch die Projektionseinrichtung 10 auf das Auge 11 abgebildet werden. Durch Triangulation und vorangegangener Kalibrierung des Systems können die 3D-Koordinaten x,y,z zu jedem Linienpixel ermittelt werden. Zur Bestimmung der Verkippung der Limbusebene werden die Teile der Linien identifiziert, die sich in unmittelbarer Nähe der Iris 23 jedoch außerhalb davon befinden. Diese Teile der Linien sind in den Figuren 4 bis 6 als die Punkte P1, P2, P3, P4 gekennzeichnet.

Zur Veranschaulichung ist in **Figur 8** der Punkt P2 als Beispiel dargestellt. Aufgrund der Anatomie des Auges liegen die Punkte P1, P2, P3, P4 in den Figuren 4 bis 6 nahezu in einer Ebene, die parallel zur Irisebene ausgerichtet ist. Dieses Vorgehen trägt der Tatsache Rechnung, dass die auf das Auge 11 projizierten Linien, bevor sie die Iris 23 erreichen, durch die Kornea 22 und das in der Kammer 26 befindliche Kammerwasser aus ihrer ursprünglichen Richtung gebrochen werden. Das Licht der Teilbereiche der Linien 19a, 19b, die auf die Iris 23 projiziert werden, passiert auf dem Weg von der Iris 23 zur Kamera 12 wiederum das in der Kammer 26 befindliche Kammerwasser und anschließend die Kornea 22, so dass es erneut zur Brechung an den beteiligten Grenzflächen kommt, also an der Grenzfläche zwischen Kammerwasser und inner Hornhautoberfläche, so wie an der Grenzfläche zwischen äußerer Hornhautoberfläche und Luft.

Würde man die Pixel der Linien, die sich auf der Iris 23 befinden, für die Triangulationsrechnungen heranziehen, zum Beispiel den Punkt P6 in Figur 8, dann würden die Ergebnisse verfälscht werden und die Ermittlung der Verkippung des Auges wäre fehlerhaft. Für diesen Fehler wären im wesentlichen zwei Faktoren verantwortlich:

a) die Linie 19a, 19b trifft aufgrund der beschrieben Brechung unter einem anderen Winkel als dem Einfallswinkel auf die Iris 23. Dies führt zu einem Fehler der Höhenkoordinate bzw. Entfernungskoordinate in z-Richtung.

b) andererseits führt die Brechung auch dazu, dass die Lateralkoordinaten x bzw. y verfälscht werden.

Dagegen werden die Linienabschnitte auf der Sklera 21 nicht gebrochen und bieten somit im erfindungsgemäßen Verfahren die Basis für die Bestimmung der Verkippung des Auges und des Limuszentrums in drei Dimensionen Die oben beschriebenen Unstetigkeiten der projizierten oder abgebildeten Linien 19a-d sind markante Merkmale, die bei der Bestimmung des Limbus herangezogen werden. Mithilfe der Punke P1, P2, P3, P4 wird eine Ebene berechnet bzw. approximiert, deren Normalvektor die Blickrichtung angibt. Aus dem Winkel, den der Normalvektor mit der z-Achse einschließt, wird der Grad der Verkippung des Auges 11 berechnet. Aus dem Differenzvektor von Blickrichtung und z-Achse ergibt sich die Verkippungsrichtung. Das Kreuzprodukt aus Blickrichtung und z-Achse definiert die Drehachse der Verkippung. D.h., es wird die absolute Ausrichtung des Auges 11 bzw. die Blickrichtung berechnet, und nicht die Ausrichtung relativ zu einem initialen Zeitpunkt. Durch die Berechnung der Ebene mittels weniger Punkte ist der Algorithmus in der Bildverarbeitungseinrichtung 14 sehr schnell.

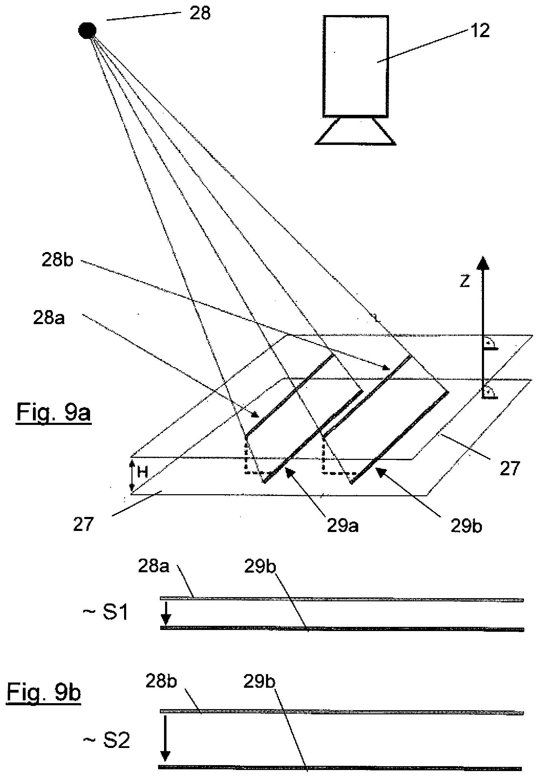

**Figur 9a und 9b** zeigen schematisch eine mögliche Kalibrierung der erfindungsgemäßen Vorrichtung zur Bestimmung der Augenposition. Zur Kalibrierung wird eine ebene Fläche 27 verwendet, die während der Kalibrierung in ihrer Höhe variiert wird. Während des Kalibriervorgangs erzeugt ein Liniengenerator 28 zwei Lichtlinien 28a, 28b auf der Fläche 27. Dabei trifft das Licht des Liniengenerators 28 zur Projektion der Lichtlinien 28a, 28b schräg bzw. unter einem Einfallswinkel auf die Fläche 27. Die Linien 28a, 28b werden von der Kamera 12 aufgenommen. In einem nächsten Schritt wird die Fläche 27 vertikal bzw. in z-Richtung um den Betrag H verschoben, dass heißt in dem gezeigten Beispiel von der Kamera 12 entfernt. Die Kameraachse muss jedoch nicht notwendigerweise mit der Verfahrrichtung der Kalibrierebene übereinstimmen. In dieser zweiten Position der Fläche 27 sind die darauf abgebildeten Lichtlinien 29a, 29b gegenüber ihrer vorhergehenden Position 28a bzw. 28b verschoben. Dabei ist die Verschiebung S1, S2 (siehe Figur 9b) der Linienposition ein Maß für den Hub H der Kalibrierebene. Die Kalibrierung ist selbstverständlich nicht nur auf Linienmuster anwendbar, sondern auch auf alle anderen Arten von projizierten Lichtmustern.

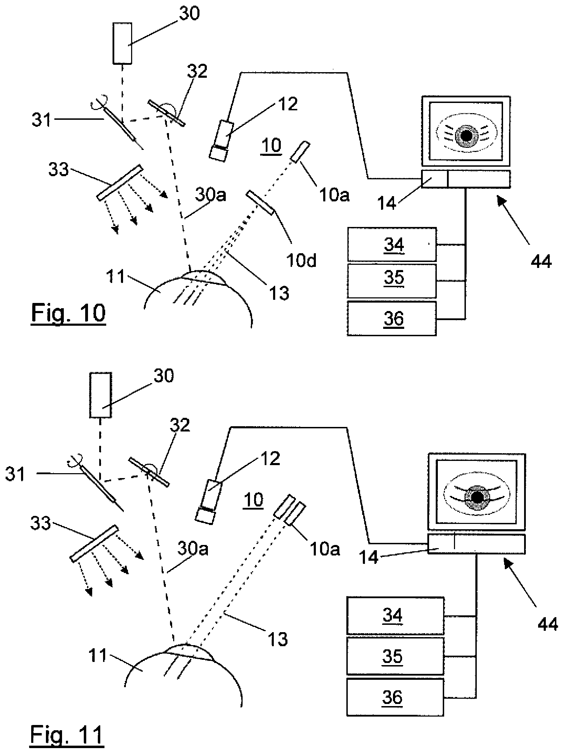

**Figur 10** zeigt eine weitere Ausführungsform der Erfindung, bei der die Projektionseinrichtung 10 einen Laser 10a oder eine LED-Einheit als Lichtquelle aufweist. Die im Zusammenhang mit Fig. 1 beschrieben Merkmale gelten auch für Fig. 10 und die nachfolgenden, weiteren Ausführungsformen. Im Strahlengang des Projektions- bzw. Lichtstrahls 13 befindet sich ein diffraktives optisches Element 10d zur Erzeugung des Lichtmusters auf dem Auge 11. Das diffraktive optische Element erzeugt beispielsweise als Lichtmuster eine Anzahl von parallelen Linien, die auf das Auge 11 projiziert werden.

Zur Beleuchtung der Szene dient eine optionale Beleuchtungseinheit 33, die vorzugsweise eine Infrarotlichtquelle ist, beispielsweise in Form einer LED Anordnung. Wenn jedoch das projizierte Lichtmuster hell genug ist, um detektiert zu werden, und das projizierte Muster bzw. die Linie den Limbus schneidet, kann auch ohne zusätzliche Beleuchtung der Limbusrand aufgrund der auftretenden Diskontinuität des Lichtmusters detektiert werden. Wenn jedoch die Position des Limbus im Bild bekannt sein muss, ist für eine ausreichende Beleuchtung zu sorgen, so dass dieser mit Mitteln der Bildverarbeitung bestimmt werden kann. Bei empfindlichen Kameras reicht jedoch oftmals auch das Umgebungslicht für die Bildaufnahme aus.

In dem hier dargestellten Beispiel ist die Vorrichtung zur Bestimmung der Augenposition an eine Laserablationseinrichtung gekoppelt, in der ein Ablationslaser 30 mittels horizontal und vertikal schwenkbaren Scan-Spiegeln 31, 32 einen Ablationslaserstrahl 30a auf die Hornhaut des Auges 11 lenkt. Eine Rechnereinheit 44 ist elektrisch mit einer Steuereinrichtung 34 zur Steuerung des Ablationslasers 30 verbunden, so wie mit einer Steuereinrichtung 35 zur Steuerung der Scan-Spiegel 31, 32 und einer Steuereinheit 36 zur Steuerung der Lichtquelle 10a, die zur Erzeugung des Lichtmusters zur Bestimmung der Augenposition dient.

Die Steuereinrichtung 34 steuert den Ablationslasers 30 derart an, dass der Ablationslaserstrahl 30a ab einer bestimmten Verkippung des Auges 11 unterbrochen wird, so dass kein Gewebeabtrag mehr stattfindet.

Die Steuereinrichtung 35 steuert die Scan-Spiegel 31, 32 derart an, dass der Ablationslaserstrahl 30a entsprechend der Verkippung des Auges nachgeführt wird. Wahlweise kann dieser aber auch auf einen Absorber gelenkt werden, damit bei z. B. zu großen Positionsabweichung zum Sollwert kein Abtrag stattfindet.

Die Steuereinrichtung 36 steuert die Lichtquelle 10a derart an, dass der Lichtstrahl zur Erzeugung des Lichtmusters bei einer zu großen Abweichung der Augenposition oder der Blickrichtung unterbunden wird. Dadurch gelangt keine Strahlung durch die Pupille in das Auge.

Es ist weiterhin möglich, mittels der Steuereinrichtung 36 die Erzeugung des Lichtmusters im Pulsbetrieb durchzuführen. Dies hat den Vorteil einer geringeren Strahlungsexposition, wenn die Mustererzeugung mit der Bilderzeugung synchronisiert wird. Das auf das Auge 11 projizierte Lichtmuster kann aber auch beispielsweise bei jedem zweiten Kamerabild erzeugt werden. In diesem Fall können über eine Differenzbildung aufeinander folgender Bilder auch intensitätsarme Muster detektiert werden.

Durch die Kopplung der erfindungsgemäßen Vorrichtung an eine Einrichtung zur Laserablation kann eine Behandlung in Echtzeit korrigiert werden. Die Schüsse oder Laserpulse werden entsprechend der jeweiligen Verkippung α des Auges nachgeführt. Somit wird der Tatsache Rechnung getragen, dass der Einfallswinkel bei jeder Verkippung des Auges vom Sollwert abweicht. Bei großen Verkippungswerten können die Spiegel 31, 32 derart angesteuert werden, dass der Laserstrahl vom Auge abgelenkt oder unterbrochen wird, um die Behandlung auszusetzen.

Der Betrag der zentralen Dezentrierung DE ergibt sich durch trigonometrische Beziehungen wie sie in Figur 2 dargestellt sind. Die Dezentrierung DE wird bestimmt durch den Verkippungswinkel sowie die Vorderkammertiefe APC. Dabei gilt:

$$DE\ /\ APC \approx \tan(\alpha).$$

Bei einem Verkippungswinkel von 7 Grad und einer Vorderkammertiefe APC von 4 mm ergibt sich der Betrag der zentralen Dezentrierung DE zu 0,5 mm. Unter Berücksichtigung der Brechungswirkung der Kornea ergibt sich für DE ein Wert von etwa 0,4mm.

Durch einen in der Figur nicht dargestellten Filter, der optional vor der Kamera 12 angebracht ist, wird die beim Laserbeschuss von dem getroffenen Gewebe emittierte Fluoreszenzstrahlung (λ<400 nm) oder auch das Umgebungslicht (λ<800 nm) blockiert. Dabei ist der Filter für Wellenlängen, die bei der Erzeugung des Lichtmusters verwendet werden, durchlässig. Somit ist die Berechnung der Verkippung des Auges sowie des Limbuszentrums unbeeinflusst von dem Ablationsvorgang und kann während diesem erfolgen.

Als Lichtquelle 10a für die Erzeugung des Lichtmusters sind alle Lichtquellen geeignet, solange die spektrale Empfindlichkeit der gewählten Kamera 12 bzw. Bilderfassungseinrichtung bei den emittierten Wellenlängen ausreichend hoch ist.

Zur Unterdrückung von Fremdlicht wird in der refraktiven Chirurgie eine Szene oft mit IR-Licht beleuchtet und ein IR-Filter platziert. In diesen Fällen ist es vorteilhaft, als Lichtquelle 10a für die Mustererzeugung ebenfalls eine IR-Quelle vorzusehen. Jedoch auch außerhalb der refraktiven Chirurgie ist es vorteilhaft eine Lichtquelle zu verwenden, deren Licht für Menschen nicht sichtbar ist und somit keinen Blendeffekt aufweist.

Der Blendeffekt kann jedoch auch dadurch vermieden werden, dass ein Muster erzeugt wird, das nicht in die Pupille gelangt und bei extremen Bewegungen ausgeschaltet wird. In der Bilderfassungseinrichtung bzw. Kamera 12 sind als Lichtsensoren vor allem flächenhafte Sensoren geeignet, die z. B digital oder analog ausgestaltet sind.

Nachfolgend werden noch weitere Ausführungsformen mit alternativen Lichtquellen 10a gezeigt. Für die Beschreibung der übrigen Elemente wird auf die vorhergehenden Figuren und zugehörige Figurenbeschreibungen Bezug genommen.

**Figur 11** zeigt eine vorteilhafte Ausführungsform der erfindungsgemäßen Vorrichtung, bei der als Lichtquelle 10a eine Anzahl von n Lasern oder LEDs mit integrierter Linienoptik verwendet werden.

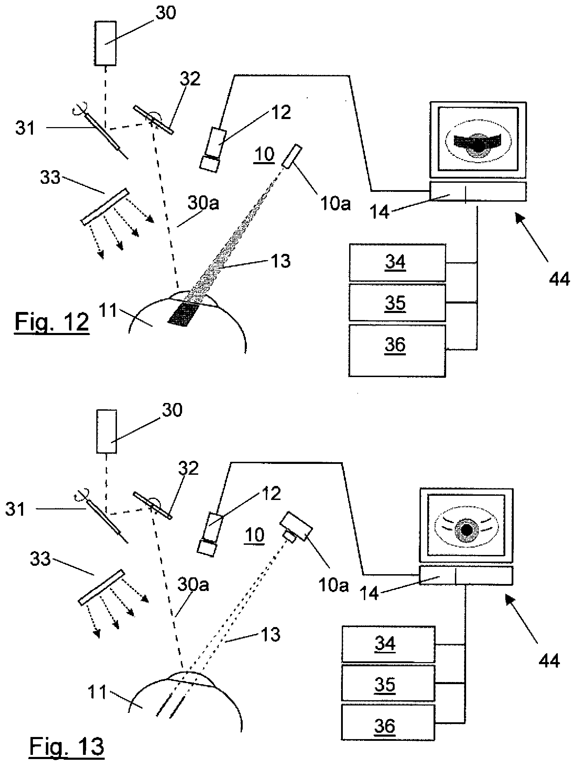

Bei der in **Figur 12** dargestellten Alternative besteht die Lichtquelle 10a aus einem Beamer oder Projektor oder einer LED bzw. Laser mit diffraktivem optischen Element. Es wird ein breiter, scharf berandeter Lichtstreifen erzeugt.

In dem in **Figur 13** gezeigten Beispiel wird ein Beamer oder Projektor als Lichtquelle 10a verwendet. Dies ist vorteilhaft besonders zur Erzeugung zeitlich veränderlicher Lichtmuster wie z. B. Linien die derart bewegt werden, dass sie stets optimal zum Limbusrand positioniert sind.

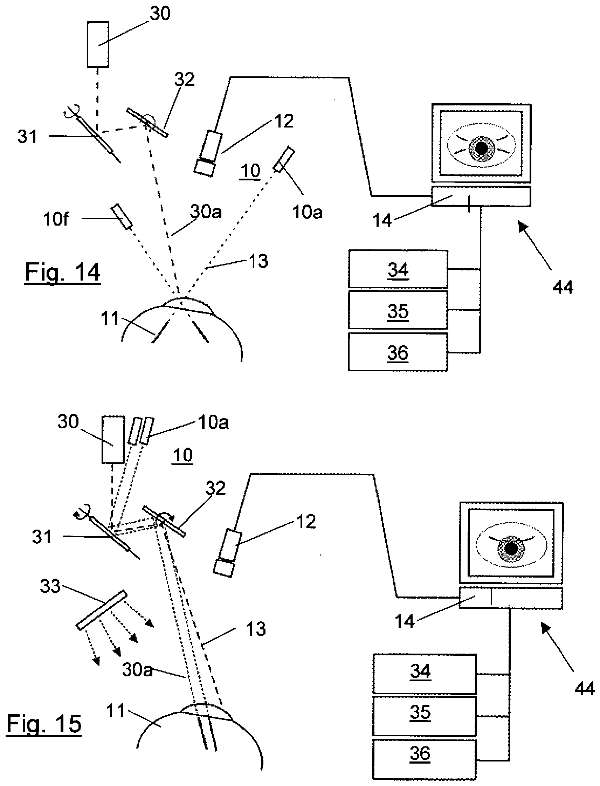

In **Figur 14** ist eine weitere Alternative gezeigt, bei der zusätzlich zur Lichtquelle 10a, die in Form einer LED oder eines Lasers mit integrierter Linienoptik ausgestaltet ist, eine weitere Lichtquelle 10f vorhanden ist, die ein Linienmuster oder sonstiges Muster aus einer anderen Richtung auf das Auge 11 projiziert.

Schließlich zeigt **Figur 15** ein weiteres Ausführungsbeispiel, bei dem die Lichtquelle 10a zur Erzeugung des Musters auf dem Auge 11 derart angeordnet ist, dass das Lichtmuster über die Scan-Spiegel 31, 32 auf das Auge 11 gelenkt wird. Die Steuereinheit 36 steuert dabei die Scan-Spiegel 31, 32 entsprechend. Die Scan-Spiegel 31, 32 können dieselben sein, über die auch der Ablationslaserstrahl 30a auf die Hornhaut des Auges 11 gelenkt wird. Dass heißt, das Lichtmuster zur Bestimmung der Augenposition wird in diesem Fall mit Hilfe der Ablenk- oder Scan-Spiegel 31, 32 des Ablationslasers 30 bewegt. Wenn die Bewegung des Musters ausreichend schnell erfolgt, dann ist prinzipiell als Muster auch nur eine Linie möglich. Zwischen zwei Kameraaufnahmen hat sich dann die Linie ausreichend weit bewegt und gleichzeitig ist die Bewegung des Auges vernachlässigbar. Es ist darauf zu achten, dass z.B. im Falle von zwei parallelen Linien nur solche Aufnahmen verwendet werden, bei denen beide Linien den Limbus "schneiden". Das muss nicht immer so sein, insbesondere wenn die Spiegel den Behandlungsstrahl auf den Randbereich der Behandlungszone lenken.

Beispielsweise könnte auch ein zwischen zwei Zuständen flippender Spiegel eine Linie auf das Auge werfen. Zwei aufeinander folgende Aufnahmen sehen dann zwei verschieden positionierte Linien. Die Bewegung sollte mit der Kamera 12 synchronisiert sein. Dadurch wird der Algorithmus vereinfacht, da pro Bild nur eine Linie emittiert werden muss.

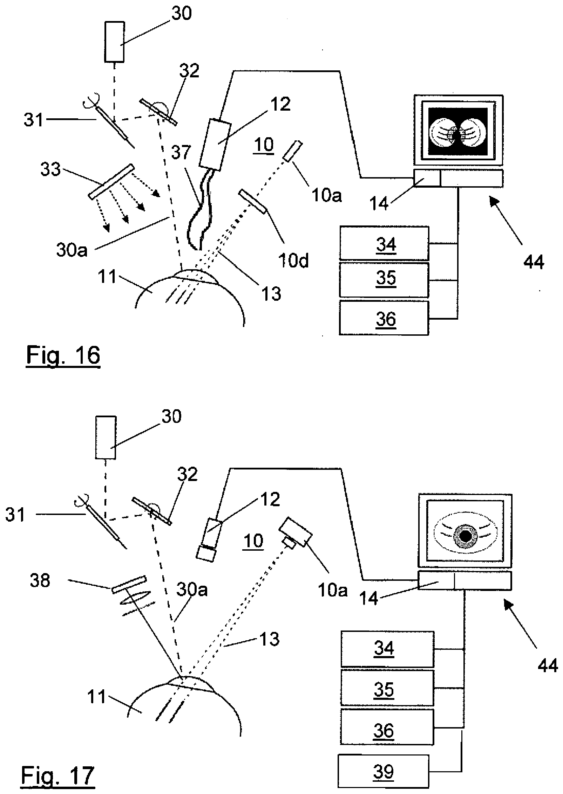

**Figur 16** zeigt zwei vor der Kamera 12 angeordnete Glasfaserbündel 37, die als Bildleiter ausgestaltet sind, über die das projizierte Muster aufgenommen wird. Dadurch kann entweder die Belichtungszeit der Kamera 12 verringert werden, oder aber die Intensität des Mustergenerators bzw. der Lichtquelle 12.

In **Figur 17** ist zusätzlich ein Fluoreszenzlichtsensor 38 bzw. PSD-Sensor mit entsprechendem Filter angeordnet, der jeden Ablationsspot anhand des emittierten Fluoreszenzlichts detektiert, und der mit einer Frequenz von mehreren kHz den Schwerpunkt seiner Projektion auf der Sensorfläche ermitteln kann. Der PSD-Sensor wird durch die Steuereinrichtung 39 gesteuert. Eine Recheneinheit ermittelt per Triangulation die 3D-Koordinaten der getroffenen Stelle. Auf diese Weise wird in Kombination mit der ermittelten Augenposition ein Echtzeit-Abtragmonitor geschaffen.

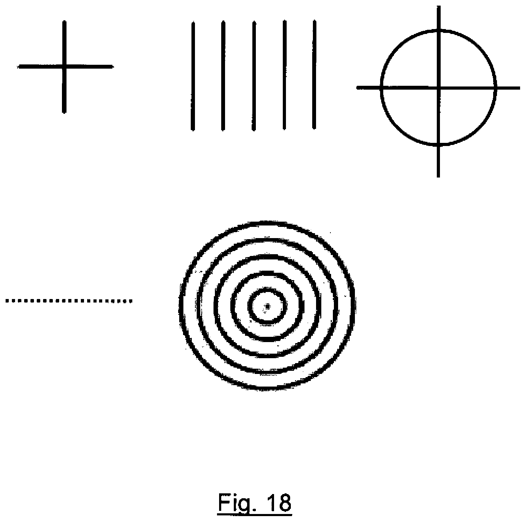

**Figur 18** zeigt mögliche Geometrien für das Lichtmuster zur Abbildung auf dem Auge, um daraus wie oben beschrieben die Verkippung des Auges um die x-Achse und / oder die y-Achse zu ermitteln und die Augenposition bzw. die Lage des Auges oder auch die Blickrichtung zu bestimmen.

Mit der vorliegenden Erfindung kann insbesondere auch der Limbus des Auges und sein Zentrum in drei Dimensionen im Raum ermittelt werden. Dies kann zusammen mit der Bestimmung der Ausrichtung der Limbus/Iris-Ebene erfolgen.

**Bezugszeichenliste**

| | |
|---|---|
| 1 | Rotationszentrum des Auges |
| 2 | Pupille |
| 2a,2b | erster und zweiter Zustand der Pupille |
| 3 | aktuelles Ablationszentrum |
| 4 | Hornhaut |
| 4a | zentraler Punkt der Hornhaut |
| 10 | Projektionseinrichtung |
| 10a | Lichtquelle |
| 10b | Doppelspalt |
| 10c | Linsensystem |
| 10d | diffraktives optisches Element |
| 11 | Auge |
| 12 | Kamera |
| 13 | Lichtstrahl |
| 14 | Bildverarbeitungseinrichtung |
| 15 | Laser |
| 16 | Lichtstreifen |
| 17 | Objekt |
| 18 | Bildsensor |
| 19a, 19b | Linien |
| 19c, 19d | Linien |
| 21 | Sklera |
| 22 | Kornea |
| 23 | Iris |
| 24 | Limbus |
| 25 | Linse |
| 26 | Kammer |
| 27 | ebene Fläche |
| 28 | Liniengenerator |
| 28a, 28b | Lichtlinien (erste Position) |
| 29a, 29b | Lichtlinien (zweite Position) |
| 30 | Ablationslaser |
| 30a | Ablationslaserstrahl |
| 31, 32 | Spiegel |
| 33 | Beleuchtungseinheit |
| 34 | Steuereinrichtung für Ablationslaser |

| 35 | Steuereinrichtung für Spiegel |
| 36 | Steuereinrichtung für Lichtquelle |
| 37 | Glasfaserbündel |
| 38 | Fluoreszenzlichtsensor |
| 39 | Steuereinrichtung für Fluoreszenzlichtsensor |
| 44 | Rechnereinheit |
| 100 | Vorrichtung |

**Patentansprüche**

1. Verfahren zur Bestimmung der Augenposition, bei dem Punkte (P1, P2, P3, P4) der Übergangsregion zwischen Sklera (21) und Iris (23) des Auges (11) ermittelt werden, deren Koordinaten berechnet werden, und daraus die Lage der Irisebene oder einer parallel dazu ausgerichteten Ebene bestimmt wird, **dadurch gekennzeichnet, dass**

   ein Lichtmuster (19a, 19b, 19c, 19d) auf eine Oberfläche des Auges (11) projiziert wird,
   wobei das Lichtmuster (19a, 19b, 19c, 19d) die Übergangsregion zwischen Sklera (21) und Iris (23) schneidet;
   und ein Bild des auf der Augenoberfläche abgebildeten Lichtmusters (19a, 19b, 19c, 19d) erfasst wird, aus dem die räumlichen Koordinaten der Punkte (P1, P2, P3, P4) der Übergangsregion zwischen Sklera (21) und Iris (23) durch Triangulation oder durch Stereovision ermittelt werden;
   wobei aus den räumlichen Koordinaten der Punkte (P1, P2, P3, P4) die Lage der Irisebene oder einer parallel dazu ausgerichteten Ebene bestimmt wird.

2. Verfahren nach Anspruch 1, **dadurch gekennzeichnet, dass** sich die Punkte auf der Sklera (21) in unmittelbarer Nähe zur Iris (23) befinden.

3. Verfahren nach einem der vorhergehenden Ansprüche, **dadurch gekennzeichnet, dass** die Punkte (P1, P2, P3, P4) aus Unstetigkeiten des erfassten Lichtmusters (19a, 19b, 19c, 19d) an der Grenze zwischen Sklera (21) und Iris (23) oder aus einem Helligkeits- und/oder Farbgradienten des Lichtmusters ermittelt werden.

4. Verfahren nach einem der vorhergehenden Ansprüche, **dadurch gekennzeichnet, dass** das auf das Auge (11) projizierte Lichtmuster (19a, 19b, 19c, 19d) aus Linienfragmenten gebildet wird, deren virtueller Schnitt mit dem Limbus des Auges (11) durch eine approximierende Funktion berechnet wird, um die Punkte (P1, P2, P3, P4) zu identifizieren.

5. Verfahren nach Anspruch 4, **dadurch gekennzeichnet, dass** zur Berechnung des virtuellen Schnitts die Linien des Lichtmusters auf der Sklera (21) und nicht im Bereich der Iris (23) abgebildet werden.

6. Verfahren nach einem der vorhergehenden Ansprüche, **dadurch gekennzeichnet, dass** das Lichtmuster (19a, 19b, 19c, 19d) unter einem Einfallswinkel auf das Auge (11) projiziert wird.

7. Verfahren nach einem der vorhergehenden Ansprüche, **dadurch gekennzeichnet, dass** das Lichtmuster (19a, 19b, 19c, 19d) aus Lichtstreifen oder Lichtringen gebildet ist, die parallel bzw. konzentrisch zueinander ausgerichtet sind.

8. Verfahren nach einem nach einem der vorhergehenden Ansprüche, **dadurch gekennzeichnet, dass** das Lichtmuster (19a, 19b, 19c, 19d) aus Lichtstreifen in Form eines Kreuzes gebildet ist.

9. Verfahren nach einem der vorhergehenden Ansprüche, **dadurch gekennzeichnet, dass** der Grad und die Richtung einer Verkippung des Auges aus dem Normalenvektor der durch die Punkte (P1, P2, P3, P4) aufgespannten Ebene ermittelt werden.

10. Verfahren nach einem der vorhergehenden Ansprüche, **gekennzeichnet durch** eine Kalibrierung, bei der das Lichtmuster (19a, 19b, 19c, 19d) auf eine ebene Fläche (27) projiziert wird, die während der Kalibrierung relativ zur Bilderfassungseinrichtung (12) verschoben wird.

11. Verfahren nach einem der vorhergehenden Ansprüche, **dadurch gekennzeichnet, dass** das Lichtmuster (19a, 19b, 19c, 19d) über das Auge (11) bewegt wird oder zeitlich veränderliche Lichtmuster (19a, 19b, 19c, 19d) erzeugt werden.

**12.** Verfahren nach einem der vorhergehenden Ansprüche, **dadurch gekennzeichnet, dass** aus dem Lichtmuster, das auf dem einen Auge eines Augenpaares erzeugt wird, die Blickrichtung oder Augenposition des anderen Auges ermittelt wird.

**13.** Verfahren nach einem der vorhergehenden Ansprüche, **dadurch gekennzeichnet, dass** sich beide Augen im Blickfeld der Kamera befinden.

**14.** Verfahren nach einem der vorhergehenden Ansprüche, **dadurch gekennzeichnet, dass** aus dem Winkel einer Verbindungslinie beider Pupillenmitten mit einer konstanten Referenzlinie eine Verdrehung des Kopfes bzw. die Kopftorsion ermittelt wird.

**15.** Vorrichtung zur Erfassung der Augenposition, umfassend:

eine Bilderfassungseinrichtung (12), und
eine Bildverarbeitungseinheit (14) zur Ermittlung mehrerer Punkte (P1, P2, P3, P4), an denen die Sklera (21) des Auges an die Iris (23) grenzt, wobei die Bildverarbeitungseinheit (14) Koordinaten der ermittelten Punkte (P1, P2, P3, P4) berechnet und daraus die Lage der Irisebene oder einer parallel dazu ausgerichteten Ebene ermittelt,
**gekennzeichnet durch**
eine Einrichtung (10) zur Projektion und Abbildung eines Lichtmusters (19a, 19b, 19c, 19d) auf einer Oberfläche des Auges (11); und
eine Bilderfassungseinrichtung (12) zur Erfassung des auf der Oberfläche des Auges (11) erzeugten Lichtmusters (19a, 19b, 19c, 19d);
wobei die Bildverarbeitungseinheit (14) aus dem erfassten Lichtmuster (19a, 19b, 19c, 19d) durch Triangulation oder durch Stereovision die räumlichen Koordinaten der Punkte (P1, P2, P3, P4) ermittelt, an denen das Lichtmuster (19a, 19b, 19c, 19d) die Übergangsregion zwischen Sklera (21) und Iris (23) schneidet, und daraus die Lage der Irisebene oder einer parallel dazu ausgerichteten Ebene ermittelt.

**16.** Vorrichtung nach Anspruch 15, **dadurch gekennzeichnet, dass** die Bildverarbeitungseinheit (14) derart gestaltet ist, dass sie die Punkte (P1, P2, P3, P4) auf der Sklera (21) in unmittelbarer Nähe zur Iris (23) ermittelt.

**17.** Vorrichtung nach Anspruch 15 oder 16, **dadurch gekennzeichnet, dass** die Bildverarbeitungseinheit (14) derart gestaltet ist, dass sie die Punkte (P1, P2, P3, P4) aus Unstetigkeiten des projizierten Lichtmusters (19a, 19b, 19c, 19d) an der Grenze zwischen Sklera (21) und Iris (23) oder aus einem Helligkeits- und/oder Farbgradienten des Lichtmusters (19a, 19b, 19c, 19d) identifiziert.

**18.** Vorrichtung nach einem der Ansprüche 15 bis 17, **dadurch gekennzeichnet, dass** die Projektionseinrichtung (10) derart gesteuert ist, dass als Lichtmuster (19a, 19b, 19c, 19d) Linienfragmente auf das Auge projiziert werden, wobei die Bildverarbeitungseinheit (14) den virtuellen Schnitt der Linienfragmente mit dem Limbus des Auges (11) durch eine approximierende Funktion berechnet, um die Punkte (P1, P2, P3, P4) zu identifizieren.

**19.** Vorrichtung nach einem der Ansprüche 15 bis 18, **gekennzeichnet durch** ein oder mehrere Glasfaserbündel (37), das vor der Bilderfassungseinrichtung (14) angeordnet ist, um das projizierte Lichtmuster (19a, 19b, 19c, 19d) auf dem Auge (11) zu erfassen.

**20.** Vorrichtung nach einem der Ansprüche 15 bis 19, **gekennzeichnet durch** eine Steuereinrichtung (35, 36) für eine Laserablationseinheit (30), die deren Ablationsstrahl (30a) bei einer Veränderung der Augenposition nachführt oder vom Auge (11) ablenkt oder unterbricht.

**21.** Vorrichtung nach einem der Ansprüche 15 bis 20, **gekennzeichnet durch** einen Fluoreszenzlicht-Sensor (38) zur Erfassung von Ablationspunkten auf dem Auge (11), die vom Ablationsstrahl (30a) getroffen wurden, wobei die Rechnereinheit die Ortskoordinaten der erfassten Ablationspunkte ermittelt.

**Claims**

1. Method for determining the eye position, in which points (P1, P2, P3, P4) of the transition region between sclera (21) and iris (23) of the eye (11) are determined, the coordinates of which are calculated, and the position of the iris plane or of a plane aligned parallel thereto is determined therefrom, **characterised in that**

   a light pattern (19a, 19b, 19c, 19d) is projected onto a surface of the eye (11), the light pattern (19a, 19b, 19c, 19d) intersecting the transition region between the sclera (21) and the iris (23);
   and an image of the light pattern (19a, 19b, 19c, 19d) displayed on the ocular surface is acquired, from which the spatial coordinates of the points (P1, P2, P3, P4) of the transition region between the sclera (21) and the iris (23) are determined by triangulation or by stereovision;
   wherein the position of the iris plane or a plane parallel thereto is determined from the spatial coordinates of the points (P1, P2, P3, P4)

2. Method according to claim 1, **characterized in that** the points on the sclera (21) are located in close proximity to the iris (23).

3. Method according to any one of the preceding claims, **characterized in that** the points (P1, P2, P3, P4) are determined from discontinuities of the detected light pattern (19a, 19b, 19c, 19d) at the boundary between the sclera (21) and the iris (23) or from a brightness and/or colour gradient of the light pattern.

4. Method according to any one of the preceding claims, **characterised in that** the light pattern (19a, 19b, 19c, 19d) projected onto the eye (11) is formed from line fragments whose virtual intersection with the limbus of the eye (11) is calculated by an approximating function to identify the points (P1, P2, P3, P4).

5. Method according to claim 4, **characterised in that** for the calculation of the virtual intersection, the lines of the light pattern are displayed on the sclera (21) and not in the area of the iris (23).

6. Method according to any one of the preceding claims, **characterized in that** the light pattern (19a, 19b, 19c, 19d) is projected onto the eye (11) at an angle of incidence.

7. Method according to any one of the preceding claims, **characterized in that** the light pattern (19a, 19b, 19c, 19d) is formed of light strips or light rings which are aligned parallel or concentric to one another.

8. Method according to any one of the preceding claims, **characterized in that** the light pattern (19a, 19b, 19c, 19d) is formed of light strips in the form of a cross.

9. Method according to any one of the preceding claims, **characterised in that** the degree and direction of a tilt of the eye are determined from the normal vector of the plane spanned by the points (P1, P2, P3, P4).

10. Method according to any one of the preceding claims, **characterised by** a calibration in which the light pattern (19a, 19b, 19c, 19d) is projected onto a planar surface (27) which is displaced relative to the image capture device (12) during the calibration.

11. Method according to any one of the preceding claims, **characterized in that** the light pattern (19a, 19b, 19c, 19d) is moved over the eye (11) or timevariable light patterns (19a, 19b, 19c, 19d) are generated.

12. Method according to any one of the preceding claims, **characterised in that** from the light pattern generated on one eye of a pair of eyes, the direction of gaze or the eye position of the other eye is determined.

13. Method according to any one of the preceding claims, **characterised in that** both eyes are in the field of view of the camera.

14. Method according to any one of the preceding claims, **characterised in that** a rotation of the head or the head torsion is determined from the angle of a connecting line of both pupil centres with a constant reference line.

15. Device for determining the eye position, comprising:

an image capture device (12), and

an image processing unit (14) for detecting a plurality of points (P1, P2, P3, P4) at which the sclera (21) of the eye is adjacent to the iris (23),

wherein the image processing unit (14) calculates coordinates of the determined points (P1, P2, P3, P4) and determines therefrom the position of the iris plane or a plane aligned parallel thereto,

**characterised by**

a device (10) for projecting and imaging a light pattern (19a, 19b, 19c, 19d) on a surface of the eye (11); and

an image capture device (12) for capturing the light pattern (19a, 19b, 19c, 19d) formed on the surface of the eye (11);

wherein the image processing unit (14) determines from the captured light pattern (19a, 19b, 19c, 19d) by triangulation or by stereovision the spatial coordinates of the points (P1, P2, P3, P4) at which the light pattern (19a, 19b, 19c, 19d) intersects the transition region between the sclera (21) and the iris (23), and determines therefrom the position of the iris plane or a plane aligned parallel thereto.

16. Device according to claim 15, **characterized in that** the image processing unit (14) is designed to determine the points (P1, P2, P3, P4) on the sclera (21) in close proximity to the iris (23).

17. Device according to claim 15 or 16, **characterized in that** the image processing unit (14) is designed to identify the points (P1, P2, P3, P4) from discontinuities of the projected light pattern (19a, 19b, 19c, 19d) at the boundary between the sclera (21) and the iris (23) or from a brightness and/or colour gradient of the light pattern (19a, 19b, 19c, 19d).

18. Device according to any one of claims 15 to 17, **characterized in that** the projection device (10) is controlled in such a way that line fragments are projected onto the eye as light patterns (19a, 19b, 19c, 19d), wherein the image processing unit (14) calculates the virtual intersection of the line fragments with the limbus of the eye (11) by an approximating function in order to identify the points (P1, P2, P3, P4).

19. Device according to any one of claims 15 to 18, **characterized by** one or more optical fibre bundles (37) arranged in front of the image capture device (14) for capturing the projected light pattern (19a, 19b, 19c, 19d) on the eye (11).

20. Device according to any one of claims 15 to 19, **characterized by** control means (35, 36) for a laser ablation unit (30), which tracks its ablation beam (30a) when the position of the eye is changed, or deflects or interrupts it from the eye (11).

21. Device according to any one of claims 15 to 20, **characterized by** a fluorescent light sensor (38) for detecting ablation points on the eye (11) which have been hit by the ablation beam (30a), the computer unit determining the location coordinates of the detected ablation points.

**Revendications**

1. Méthode pour la détermination de la position de l'oeil dans laquelle on détermine des points (P1, P2, P3, P4) de la zone de transition située entre la sclérotique (21) et l'iris (23) de l'oeil (11), on calcule leurs coordonnées et on détermine à partir de là la position du plan de l'iris ou d'un plan orienté parallèlement à ce plan, **caractérisée en ce que**

un motif lumineux (19a, 19b, 19c, 19d) est projeté sur une surface de l'oeil (11),

le motif lumineux (19a, 19b, 19c, 19d) coupant la zone de transition située entre la sclérotique (21) et l'iris (23) ; et une image du motif lumineux (19a, 19b, 19c, 19d) reproduit sur la surface de l'oeil est saisie, image à partir de laquelle les coordonnées spatiales des points (P1, P2, P3, P4) de la zone de transition située entre la sclérotique (21) et l'iris (23) sont déterminées par triangulation ou par stéréovision ;

la position du plan de l'iris ou d'un plan orienté parallèlement à celui-ci étant déterminée à partir des coordonnées spatiales des points (P1, P2, P3, P4).

2. Méthode selon la revendication 1, **caractérisée en ce que** les points sur la sclérotique (21) sont situés à proximité immédiate de l'iris (23).

3. Méthode selon l'une quelconque des revendications précédentes, **caractérisée en ce que** les points (P1, P2, P3, P4) sont déterminés à partir de discontinuités du motif lumineux saisi (19a, 19b, 19c, 19d) à la limite entre la sclérotique (21) et l'iris (23) ou à partir d'un gradient de luminosité et/ou de couleur du motif lumineux.

4. Méthode selon l'une quelconque des revendications précédentes, **caractérisée en ce que** le motif lumineux (19a, 19b, 19c, 19d) projeté sur l'oeil (11) est formé de fragments de lignes dont l'intersection virtuelle avec le limbe de l'oeil (11) est calculée par une fonction d'approximation afin d'identifier les points (P1, P2, P3, P4).

5. Méthode selon la revendication 4, **caractérisée en ce que**, pour le calcul de la coupe virtuelle, les lignes du motif lumineux sont représentées sur la sclérotique (21) et non dans la zone de l'iris (23).

6. Méthode selon l'une quelconque des revendications précédentes, **caractérisée en ce que** le motif lumineux (19a, 19b, 19c, 19d) est projeté sur l'oeil (11) sous un angle d'incidence.

7. Méthode selon l'une quelconque des revendications précédentes, **caractérisée en ce que** le motif lumineux (19a, 19b, 19c, 19d) est formé de bandes ou d'anneaux lumineux qui sont alignés parallèlement ou concentriquement les uns aux autres.

8. Méthode selon l'une quelconque des revendications précédentes, **caractérisée en ce que** le motif lumineux (19a, 19b, 19c, 19d) est formé de bandes lumineuses en forme de croix.

9. Méthode selon l'une quelconque des revendications précédentes, **caractérisée en ce que** le degré et la direction d'un basculement de l'oeil sont déterminés à partir du vecteur normal du plan défini par les points (P1, P2, P3, P4).

10. Méthode selon l'une quelconque des revendications précédentes, **caractérisée par** un étalonnage dans lequel le motif lumineux (19a, 19b, 19c, 19d) est projeté sur une surface plane (27) qui est déplacée pendant l'étalonnage par rapport au dispositif de saisie d'images (12).

11. Méthode selon l'une quelconque des revendications précédentes, **caractérisée en ce que** le motif lumineux (19a, 19b, 19c, 19d) est déplacé sur l'oeil (11) ou des motifs lumineux pouvant varier dans le temps (19a, 19b, 19c, 19d) sont générés.

12. Méthode selon l'une quelconque des revendications précédentes, **caractérisée en ce que** la direction du regard ou la position de l'autre oeil est déterminée à partir du motif lumineux qui est généré sur l'un des yeux d'une paire d'yeux.

13. Méthode selon l'une quelconque des revendications précédentes, **caractérisée en ce que** les deux yeux se trouvent dans le champ de vision de la caméra.

14. Méthode selon l'une quelconque des revendications précédentes, **caractérisée en ce qu'**une rotation de la tête ou la torsion de la tête est déterminée à partir de l'angle d'une ligne de liaison des deux centres de pupille avec une ligne de référence constante.

15. Dispositif pour la saisie de la position de l'oeil, comprenant :

   un dispositif de saisie d'images (12), et
   une unité de traitement d'images (14) pour la détermination de plusieurs points (P1, P2, P3, P4) sur lesquels la sclérotique (21) de l'oeil est adjacente à l'iris (23),
   l'unité de traitement d'images (14) calculant les coordonnées des points déterminés (P1, P2, P3, P4) et déterminant à partir de là la position du plan de l'iris ou d'un plan orienté parallèlement à celui-ci,
   **caractérisé par**
   un dispositif (10) pour la projection et la représentation d'un motif lumineux (19a, 19b, 19c, 19d) sur une surface de l'oeil (11) ; et
   un dispositif de saisie d'images (12) pour la saisie du motif lumineux (19a, 19b, 19c, 19d) généré sur la surface de l'oeil (11) ;
   l'unité de traitement d'images (14) déterminant à partir du motif lumineux (19a, 19b, 19c, 19d) saisi, par triangulation ou par stéréovision, les coordonnées spatiales des points (P1, P2, P3, P4) sur lesquels le motif lumineux (19a, 19b, 19c, 19d) coupe la zone de transition entre la sclérotique (21) et l'iris (23), et déterminant à partir de là la position du plan de l'iris ou d'un plan orienté parallèlement à celui-ci.

**16.** Dispositif selon la revendication 15, **caractérisé en ce que** l'unité de traitement d'images (14) est conçue pour déterminer les points (P1, P2, P3, P4) sur la sclérotique (21) à proximité immédiate de l'iris (23).

**17.** Dispositif selon la revendication 15 ou 16, **caractérisé en ce que** l'unité de traitement d'images (14) est conçue pour identifier les points (P1, P2, P3, P4) à partir de discontinuités du motif lumineux projeté (19a, 19b, 19c, 19d) à la limite entre la sclérotique (21) et l'iris (23) ou à partir d'un gradient de luminosité et/ou de couleur du motif lumineux (19a, 19b, 19c, 19d).

**18.** Dispositif selon l'une quelconque des revendications 15 à 17, **caractérisé en ce que** le dispositif de projection (10) est commandé de telle sorte que des fragments de ligne sont projetés sur l'oeil en tant que motifs lumineux (19a, 19b, 19c, 19d), l'unité de traitement d'images (14) calculant l'intersection virtuelle des fragments de ligne avec le limbe de l'oeil (11) par une fonction d'approximation afin d'identifier les points (P1, P2, P3, P4).

**19.** Dispositif selon l'une quelconque des revendications 15 à 18, **caractérisé par** un ou plusieurs faisceaux de fibres optiques (37) disposés devant le dispositif de saisie d'images (14) afin de saisir le motif lumineux projeté (19a, 19b, 19c, 19d) sur l'oeil (11).

**20.** Dispositif selon l'une quelconque des revendications 15 à 19, **caractérisé par** un dispositif de commande (35, 36) pour une unité d'ablation au laser (30) qui suit le faisceau d'ablation (30a) de celle-ci lors d'un changement de la position de l'oeil ou le dévie de l'oeil (11) ou l'interrompt.

**21.** Dispositif selon l'une quelconque des revendications 15 à 20, **caractérisé par** un capteur de lumière fluorescente (38) pour la saisie des points d'ablation sur l'oeil (11) qui ont été frappés par le faisceau d'ablation (30a), l'unité de calcul déterminant les coordonnées locales des points d'ablation saisis.

**Fig. 1**

Fig. 2

Fig. 3

Fig. 4a

Fig. 4b

Fig. 5a

Fig. 5b

11  19a                            19b

P1 ○          ○ P2

21                              23

19b                             19a

P3 ○          ○ P4

24

**Fig. 5c**

19a                            19b

11

21                              23

19c                             19d

P1      P2

P3      P4

24

**Fig. 6**

Fig. 7

19a

Fig. 8

Fig. 9a

Fig. 9b

EP 2 582 284 B1

Fig. 10

Fig. 11

30

31    32
              12
          10
33         10a    14
       30a              44
              13
   11
Fig. 12

34
35
36

30

31    32
              12
          10
33            10a  14
       30a              44
              13
   11
Fig. 13

34
35
36

Fig. 14

Fig. 15

Fig. 16

Fig. 17

Fig. 18

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102004020356 A1
- DE 19954047 B4
- DE 102008049846 A1
- DE 102008039838 A1
- US 4761071 A
- US 5471542 A
- WO 0134021 A1
- DE 102006002001 A1
- WO 0027273 A1
- WO 9918868 A1
- DE 19719695 A1
- US 2006215111 A1
- US 6019755 A
- US 2001022648 A1
- WO 0189373 A2
- DE 102004025999 A1